# EUROPEAN PATENT SPECIFICATION

(11) **EP 3 983 832 B1**
(45) Date of publication and mention of the grant of the patent: **14.08.2024**
(21) Application number: 20751765.7
(22) Date of filing: 12.06.2020
(51) Int. Cl.: G01V 9/00

(54) **METHOD FOR DETERMINING PRESENCE OF RESERVOIRED HYDROCARBONS**
VERFAHREN ZUR BESTIMMUNG DER PRÄSENZ VON AUFGESPEICHERTEN KOHLENWASSERSTOFFEN
PROCÉDÉ POUR DÉTERMINER LA PRÉSENCE D'HYDROCARBURES EN RÉSERVOIRS

(30) Priority: 13.06.2019 US 201962860827 P; 09.07.2019 EP 19185139
(43) Date of publication of application: 20.04.2022
(73) Proprietor: Shell Internationale Research Maatschappij B.V., 2596 HR The Hague (NL)
(72) Inventor: BAKSMATY, Leslie Owuraku, Houston, Texas 77082 (US); RATNAKAR, Ram Ratan, Houston, Texas 77082 (US); DINDORUK, Birol, Houston, Texas 77082 (US)
(74) Representative: Shell Legal Services IP
(86) International application number: PCT/US2020/037411
(87) International publication number: WO 2020/252245

(56) References cited:
- US-A1- 2006 154 306
- US-A1- 2008 147 326
- US-A1- 2016 341 038
- US-A1- 2018 321 215
- SUSAN MAU ET AL: "Compositional variability and air-sea flux of ethane and propane in the plume of a large, marine seep field near Coal Oil Point, CA", GEO-MARINE LETTERS ; AN INTERNATIONAL JOURNAL OF MARINE GEOLOGY, SPRINGER, BERLIN, DE, vol. 30, no. 3-4, 20 February 2010 (2010-02-20), pages 367 - 378, XP019845758, ISSN: 1432-1157
- THOMAS PAPE ET AL: "Gas hydrates in shallow deposits of the Amsterdam mud volcano, Anaximander Mountains, Northeastern Mediterranean Sea", GEO-MARINE LETTERS ; AN INTERNATIONAL JOURNAL OF MARINE GEOLOGY, SPRINGER, BERLIN, DE, vol. 30, no. 3-4, 16 March 2010 (2010-03-16), pages 187 - 206, XP019845769, ISSN: 1432-1157
- G. ETIOPE ET AL: "Emission of Methane and Heavier Alkanes From the La Brea Tar Pits Seepage Area, Los Angeles : Gas seepage at La Brea Tar Pits", JOURNAL OF GEOPHYSICAL RESEARCH: ATMOSPHERES, vol. 122, no. 21, 11 November 2017 (2017-11-11), pages 12,008 - 12,019, XP055665258, ISSN: 2169-897X, DOI: 10.1002/2017JD027675

## Description

### FIELD OF THE INVENTION

The present invention relates to the field of determining the presence of reservoired hydrocarbons from a hydrocarbon seep.

### BACKGROUND OF THE INVENTION

Exploration for reservoired hydrocarbons in a marine environment is more costly and complex compared to many exploration endeavours on land. An important tool for improving the probability of success in discovering new oil reserves is detecting seeps, effectively leaks of hydrocarbons from reservoirs. However, not all seeps are indicative of reservoired hydrocarbons. In some instances, seeps are not connected to reservoired hydrocarbons.

A method for detecting hydrocarbon seepages into the sea is described in US9,612,231B2 (Pottorf et al., 4 Apr 2017). The method starts with performing a remote sensing survey and analysing the remote sensing data from the remote sensing survey to determine the location of hydrocarbon seeps into the sea. The remote sensing survey may include performing one or more of ocean acoustic waveguide survey, water column seismic survey, active acoustic sensing survey, imagery and spectrometry of slicks and atmospheric gas plumes, passive acoustic sensing survey, magnetic and gravity surveys, optical sensing survey and thermal anomalies detection survey. These surveys include seismic and acoustic imaging of seeps in the water column, performed in ship-based marine vessels, using multibeam echo sounder and/or side-scan sonar.

In another member of the same patent family, US20140250999A1 (Lawson et al., 11 Sept 2014) describes a method for reservoir surveillance. Samples of produced fluids are analysed for changes over time in noble gas and clumped isotope signatures. And in yet another member of the same family, US20150127313A1 (Lawson et al., 7 May 2015) describes a method for determining the presence and location of a subsurface hydrocarbon accumulation by comparing a clumped isotopic signature with an expected or theoretical concentration of isotopologues of a hydrocarbon species calculated by molecular modelling. According to Lawson et al, the *"differentiation between direct seepage from a source rock from the leakage of hydrocarbons from a subsurface accumulation requires consideration of the clumped isotopic signatures that may result from the two models of seepage. Hydrocarbons that have migrated directly from a source rock may either (i) retain a stochastic clumped isotope signature given insufficient time for a thermal contribution to the "clumping" of multiply substituted isotopologues, or (ii) display an inconsistent clumped isotope signature that arises as a result of the variability in the rate of isotope exchange of individual isotopologues. In contrast, hydrocarbons that derive from a subsurface accumulation will retain a clumped isotope signature that more consistently reflects the temperature at which they were stored in the subsurface."* The solution presented in Lawson et al is to calculate a theoretical clumped isotopic signature for each isotopologue using molecular modelling.

Kennicutt et al ("Leakage of deep, reservoired petroleum to the near surface on the Gulf of Mexico continental slope," Marine Chemistry 24:39-59; 1988) discusses the link between natural seepage in a deepwater marine setting and the formation of sea slicks and tar balls. Analysis of the gaseous and liquid hydrocarbons show that gas migrates to shallow sediments with little or no isotopic fractionation. Table 1 illustrates that carbon isotopic compositions are essentially unchanged after migration from reservoirs at depths > 2000 m. In contrast, Kennicutt et al found that near-surface hydrocarbon liquids were depleted in aliphatics, 4-ring or larger aromatics, naphthalene, C1-naphthalenes and C2-naphthalenes as compared to reservoired fluids.

Sassen et al ("Massive vein-filling gas hydrate: relation to ongoing gas migration from the deep subsurface in the Gulf of Mexico," Marine and Petroleum Geology 18:551-560; 2001) show correlation of isotopic properties of C1-C5 hydrocarbons from reservoirs, gas vents and gas hydrates for the same seeps in Kennicutt et al (1988).

AbuAli et al. (US2016/0341038A1, 24 Nov 2016) describes a sampling technique used to detect hydrocarbon microseeps in a subterranean formation by inserting probes into multiple different sampling depths from the surface.

Peterson et al. (US2018/0321215A1, 8 Nov 2018) discloses a method for determining a characteristic of a hydrocarbon source by analyzing a hydrocarbon fluid sample to determine a measure clumped isotope signature and comparing to an expected clumped isotope signature.

Ellis (US2008/0147326A1, 19 Jun 2008) relates to a system and method for utilizing acquired compositional and isotopic data for an interpretive method of mud gas isotope logging to determine hydrocarbon charge, source identification, maturity, reservoir hydrocarbon isotopic signature, good hydrocarbon communication, seals, baffles, or other barriers to hydrocarbon communication in oil and gas drilling prospects.

There is a need for methods to determine if a seep is more likely to be connected to reservoired hydrocarbons. There is also a need for classifying a plurality of seeps as originating from the same or different reservoir.

### SUMMARY OF THE INVENTION

According to one aspect of the present invention, there is provided a method for determining a presence of reservoired hydrocarbons having a hydrocarbon seep, the method comprising the steps of: (a) locating a hydrocarbon seep at a seabed location where hydrocarbon is actively flowing out of the seabed; (b) determining at least two temporally spaced molecular compositions of the hydrocarbon seep; (c) determining a temporal variance between the at least two temporally spaced molecular compositions; (d) when the temporal variance falls within a predetermined temporal tolerance, classifying the hydrocarbon seep as being indicative of the presence of reservoired hydrocarbons; and (e) assigning a unique identifier to the reservoired hydrocarbons.

According to another aspect of the present invention, there is provided a method for determining a unique hydrocarbon reservoir having a hydrocarbon seep, the method comprising the steps of: (a) locating a plurality of hydrocarbon seeps at a seabed location where hydrocarbon is actively flowing out of the seabed; (b) determining at least two temporally spaced molecular compositions for each of the plurality of hydrocarbon seeps; (c) determining a temporal variance between the at least two temporally spaced molecular compositions for each of the plurality of hydrocarbon seeps; (d) when the temporal variance for any one of the plurality of hydrocarbon seeps falls within a predetermined temporal tolerance, classifying the hydrocarbon seep as being indicative of a presence of a reservoired hydrocarbons; (e) selecting a representative molecular composition for each of the plurality of hydrocarbon seeps classified as being indicative of the presence of reservoired hydrocarbons; (f) determining a spatial variance between the representative molecular compositions; (g) for the hydrocarbon seeps having the spatial variance falling within a predetermined spatial tolerance, assigning a unique identifier to the subsurface hydrocarbon accumulation; and (h) for the one or more hydrocarbon seeps wherein the spatial variance falls outside the predetermined spatial tolerance, assigning one or more additional unique identifiers to the reservoired hydrocarbons.

### BRIEF DESCRIPTION OF THE DRAWINGS

The present invention will be better understood by referring to the following detailed description of preferred embodiments and the drawings referenced therein, in which:
Fig. 1 illustrates different types of hydrocarbon seeps in a body of water; and
Fig. 2 illustrates a hydrocarbon reservoir with 2 active hydrocarbon seeps.

### DETAILED DESCRIPTION OF THE INVENTION

In one embodiment, the present invention provides a method for determining a presence of reservoired hydrocarbons having a hydrocarbon seep. In another embodiment, the method of the present invention provides a method for determining a unique hydrocarbon reservoir having a plurality of hydrocarbon seeps.

The methods of the present invention involve determining at least two temporally spaced molecular compositions from a hydrocarbon seep. By determining whether a variance between two temporally spaced molecular compositions falls within a temporal tolerance, the method of the present invention determines that a steady state exists, thereby suggesting that the hydrocarbon seep is more likely indicative of reservoired hydrocarbons, as opposed to a seep originating directly from a source rock.

"Reservoired hydrocarbons" as used herein means that the hydrocarbons are reservoired in an oil reservoir or a gas reservoir. A reservoir is an underground formation containing an individual and separate natural accumulation of producible hydrocarbons. An oil reservoir generally contains gas, oil and water, with oil being a major component. When gas accumulates independently of the oil, the reservoir is referred to as a gas reservoir, which may also contain some water and oil. A gas reservoir is a naturally occurring storage area of natural gas. In a condensate reservoir, hydrocarbons may exist as a gas, but when brought to the surface, the heavier hydrocarbons condense to a liquid.

A "seep" or "hydrocarbon seep" is an indicator of hydrocarbons escaping the subsurface to a seabed, often under low pressure or flow. The hydrocarbons may escape from a reservoir along geological layers, or through fractures and fissures in the rock. Hydrocarbon seeps may result in bubble plumes in a water column, production of gas hydrates on the seabed, and/or production of oil slicks on the surface of the sea. Seeps may also arise from hydrocarbons escaping source rock that are not connected to a reservoir.

As used herein, "actively flowing" means that there is mass transfer of hydrocarbons. Indicators of an actively flowing seep include, without limitation, a bubble plume, a distinct phase from surrounding water, intrusion of one fluid into another, non-diffusive transport, and an accumulation of gas hydrates or oil that increases with time. The term "bubbles" is used for vapour-filled bubbles, as well as liquid droplets. The bubbles may or may not be at least partially frozen. In the case of hydrocarbon-containing bubbles, the bubbles may be at least partially in the form of hydrocarbon hydrates, such as methane hydrates. For example, a bubble may have a hydrate shell around a hydrocarbon fluid. Accordingly, it will be understood by those skilled in the art that the bubble plume may be formed of liquid or gas depending on, for example, the depth of the water. Often, in a subsea hydrocarbon seep, C₁ - C₄ components, which are typically gaseous at STP, are in liquid form at or near the seabed.

As used herein, "isotopes" refers to variants of a chemical element with different numbers of neutrons. For example, carbon has 15 known isotopes, from ⁸C to ²²C, of which ¹²C and ¹³C are stable isotopes. Hydrogen has three-naturally occurring isotopes - protium ¹H with zero neutrons, deuterium ²H (or D) with one neutron and tritium ³H with two neutrons. "Isotopologues" are molecules that have the same chemical composition but differ only in their isotopic composition. As an example, methane has ten stable isotopologues: ¹²CH₄, ¹³CH₄, ¹²CH₃D, ¹³CH₃D, ¹²CH₂D₂, ¹²CH₂D₂, ¹³CH₂D₂, ¹²CHD₃, ¹³CHD₃, ¹²CD₄ and ¹³CD₄. In this example, ¹²CH₄ is an unsubstituted isotopologue, ¹³CH₄ and ¹²CH₃D are singly substituted isotopologues and ¹³CH₃D and ¹²CH₂D₂ are doubly substituted isotopologues. Multiple-substituted isotopologues, for example ¹³CH₃D and ¹²CH₂D₂, are termed "clumped isotopologues."

By "isotopic composition," we mean the relative amounts or distribution of individual isotopologues measured (in wt. or mol%).

By "molecular composition," we mean the relative amounts or distribution of individual molecules measured (in wt. or mol%). The molecular composition may be limited to hydrocarbons, for example C1-C7 hydrocarbons, or may also include non-hydrocarbons of interest.

As used herein, "steady state" means that the molecular, and, preferably isotopic, composition is stable with respect to time, within a predetermined tolerance.

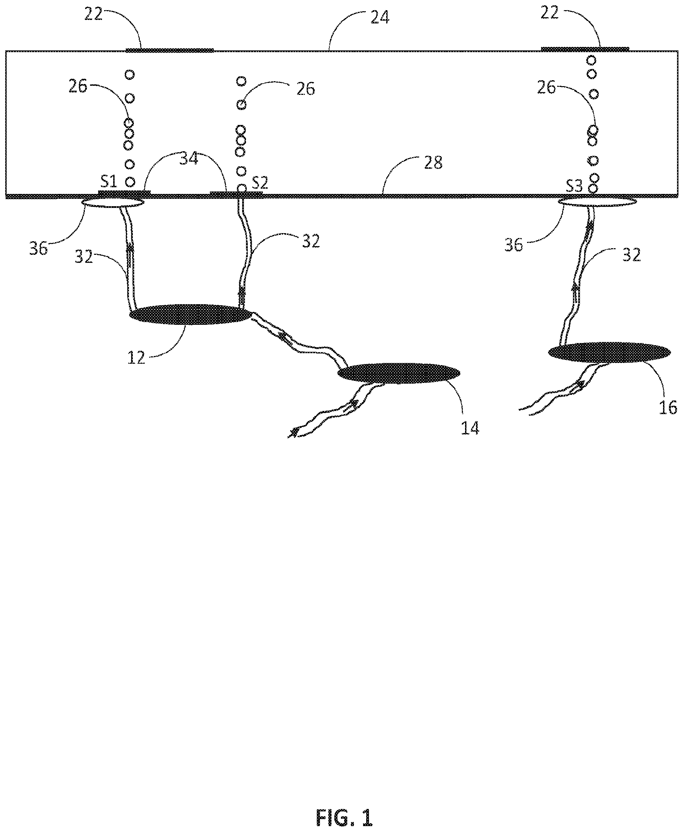

Referring now to the drawing, Fig. 1 illustrates different types of hydrocarbon seeps from hydrocarbon reservoirs 12, 14, 16. Oil slicks 22 at the sea surface 24 or hydrocarbon streams 26, as a bubble plume and/or a distinct phase from surrounding water, at the sea surface 24 or seabed 28 may be indicative of a subsurface reservoir 12, 14, 16. Hydrocarbons from a hydrocarbon reservoir 12, 14, 16 migrate to the seabed 28 through fractures 32. At the seabed 28, microbial, especially bacterial, mats 34 and/or other sea life (not shown), such as tube worms and seep mussels, may accumulate around a seep location, S1, S2, S3. However, the microbial mats 34 may not necessarily be visible or present around an active seep S1, S2, S3. Furthermore, microbial mats 34 may form at other hydrocarbon sources (not shown) that do not originate from a hydrocarbon reservoir 12, 14, 16.

For simplicity, oil slicks 22 and hydrocarbon streams 26 are illustrated as being located directly above the seep S1, S2, S3. However, it will be understood by those skilled in the art that the oil slicks 22 and hydrocarbon streams 26 may drift with wave and/or motion and/or tidal forces at and/or below the sea surface 24 and may be some distance from the seep S1, S2, S3. Fig.1 depicts gas accumulations 36 at or below the seabed 28. Typically, the gas accumulations 36 are associated with a reservoir 12, 14, 16. The gas accumulations 36 may be in the form of trapped gas and/or gas hydrates. For ease of discussion, the various elements in Fig. 1 are not to scale. For example, the gas accumulation 36 may be significantly smaller than the reservoir 12, 14, 16.

Fig. 1 illustrates one active seep S3 from hydrocarbon reservoir 16. Fig. 1 also shows a reservoir 14 that is connected to another reservoir 12. When reservoir 12 is well-mixed, the composition from seep S1 and seep S2 will be substantially similar. However, when reservoir 12 is partitioned, the composition from seep S1 and seep S2 may be different. Isotopic compositions from seep S1 and seep S2 are more likely to be substantially similar. The molecular composition from seep S1 may differ from seep S2 due to partitioning effects in reservoir 12 and/or gas accumulation 36.

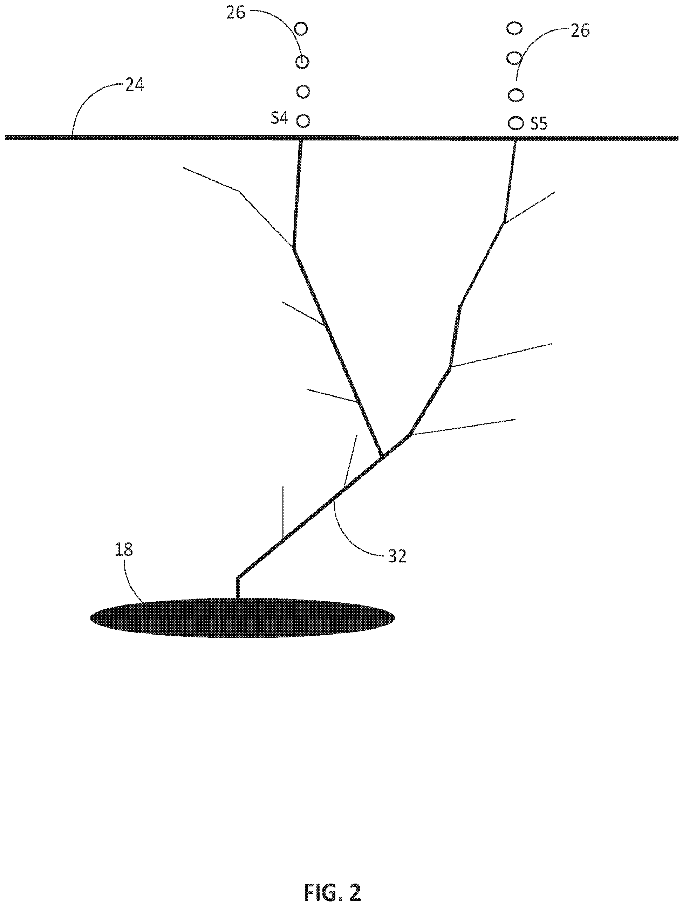

Fig. 2 illustrates an embodiment where seeps S4 and S5 originate from the same reservoir 18 from fracture 32, which later splits into two main fractures resulting in separate seeps S4 and S5. Isotopic and/or molecular compositions of hydrocarbon streams 26 are expected to be substantially similar for seeps S4 and S5. For ease of discussion, the various elements in Fig. 2 are not to scale.

In accordance with the present invention, a hydrocarbon seep S1, S2, S3, S4, S5 is first found at a seabed location where hydrocarbon is actively flowing out of the seabed 28. The hydrocarbon seep S1, S2, S3, S4, S5 may be detected for example, without limitation, by satellite, aircraft or watercraft detection and/or observation of hydrocarbon slicks 22 and/or hydrocarbon streams 26. The hydrocarbon seep S1, S2, S3, S4, S5 may also be located by planned or unplanned surveillance by an underwater vehicle.

Once located, at least two temporally spaced molecular compositions of the hydrocarbon seep S1, S2, S3, S4, S5 are determined. The molecular compositions are determined for example, without limitation, by gas chromatography-mass spectrometry (GC/MS), GC/GC/MS, and/or liquid chromatography. In another embodiment, analysis of samples may also be conducted. This may include, for example, without limitation, inductively coupled plasma mass spectrometry (ICP-MS) and ICP-optical emission spectroscopy. Gas chemistry analysis may also be conducted and may include isotope ratio-mass spectrometry and GC.

Preferably, at least two temporally spaced isotopic compositions of the hydrocarbon seep S1, S2, S3, S4, S5 are determined. The isotopic compositions are determined, for example, without limitation, by mass spectroscopy, laser-based spectroscopy, and other methods/apparatus known to those skilled in the art. The isotopic compositions are determined, for example, without limitation, by mass spectroscopy, laser-based spectroscopy, and other methods/apparatus known to those skilled in the art. Preferably, the temporally spaced isotopic composition is defined by isotopic compositions of hydrocarbons selected from the group consisting of methane, ethane, propane, butane, and combinations thereof. More preferably, the temporally spaced isotopic composition is defined by isotopic compositions of hydrocarbons, such as, for example, without limitation, methane, ethane, propane, and/or butane, as well as isotopic compositions of non-hydrocarbons, such as, for example, without limitation, carbon dioxide and/or hydrogen sulphide.

Preferably, the compositions are determined by capturing and/or in-situ probing hydrocarbons from the hydrocarbon streams 26. For example, a sample of hydrocarbon stream 26 may be captured isobarically, so that the components of the sample remain unchanged when taken to a testing site, for example at sea level. Alternatively, compositions may be determined by underwater mass spectrometers, Raman spectroscopy, isotope probes, and the like.

Samples may be obtained, for example, by divers, underwater vehicles, including manned and unmanned submersibles, remotely operated underwater vehicles (ROV), autonomous underwater vehicles (AUV), and the like. Alternatively, or in combination, the compositions may be determined without the need for a physical sample, for example, by Raman spectroscopy or isotope probes, known to those skilled in the art.

Analysis of the samples may be determined in-situ and/or remotely.

A camera may be used while collecting samples. In a preferred embodiment samples of hydrocarbon gas and hydrocarbon oil are taken from the same seep location. The gas and oil may be captured in the same sample container or may be independently captured. In the latter case, it may be particularly advantageous to use a camera to determine the volumetric flow rate. In this way, the gas and oil samples may be combined in the correct volumetric ratio for an analysis of the recombined sample.

The at least two temporally spaced molecular compositions may be determined, for example, by compositions may be determined by samples and/or measurements taken 12 to 24 hours apart. Likewise, the at least two temporally spaced isotopic compositions may be determined, for example, by samples taken 12 to 24 hours apart. The objective of temporal spacing is to account for periodicity, especially, for example, tidal forces, to assess whether a composition is at steady state. Accordingly, it is within the scope of the present invention to determine a composition at time to and then determine a composition 1 day or week, for example, later, with or without an offset of 12 hours.

The at least two temporally spaced molecular, and, preferably isotopic compositions of the hydrocarbon seep are analysed for relative amounts or distribution of certain or all molecular, and, preferably isotopic components. Preferably, more than two temporally spaced molecular, and, preferably isotopic compositions of the hydrocarbon seep are determined and analysed to improve resolution of the compositions.

While molecular compositions may be susceptible to diffusivity effects, substantial consistency between temporally spaced molecular compositions is a strong indicator of the presence of a hydrocarbon accumulation.

Isotopic compositions are particularly useful for identifying the presence of a hydrocarbon accumulation because they are indicative of the thermal maturity of the hydrocarbon accumulation. Furthermore, isotopic compositions are substantially unaffected by diffusivity as the hydrocarbon migrates to the seabed. Accordingly, in a preferred embodiment of the present invention, both molecular and isotopic compositions are determined.

Oil samples typically will have a strong relationship to the source of reservoired hydrocarbons. Gas samples are more sensitive to how the gas migrated to the seabed from the reservoir.

In a preferred embodiment, a base set of isotopologues and/or compounds are selected for comparing temporal and/or spatial variances between temporally spaced compositions.

Nonhydrocarbon gases, such as hydrogen sulphide and carbon dioxide, may be produced with or in addition to hydrocarbons. Preferably, the nonhydrocarbon gases are considered as part of the molecular, and, preferably isotopic composition. More preferably, CO2 is considered as part of the molecular, and, preferably isotopic composition. The information about carbon dioxide can provide more insights into the reservoir characteristics.

The at least two temporally spaced compositions are then compared to determine a temporal variance between the compositions. When the temporal variance falls within a predetermined temporal tolerance, the hydrocarbon seep related to the at least two compositions is classified as being indicative of the presence of reservoired hydrocarbons. The value of the temporal tolerance will be dependent on a number of factors, including the compound and/or isotope/isotopologue being considered, the time difference between samples, tidal forces, and the like.

When a hydrocarbon seep is classified as being indicative of the presence of reservoired hydrocarbons, a unique identifier can be assigned to the reservoired hydrocarbons.

In one embodiment, the method involves locating a plurality of hydrocarbon seeps. At least two temporally spaced compositions are determined and when a temporal variance between the at least two temporally spaced compositions falls within a predetermined temporal tolerance, a hydrocarbon seep is classified as being indicative of the presence of reservoired hydrocarbons. A representative composition is then selected for each of the hydrocarbon seeps classified as being indicative of the presence of reservoired hydrocarbons. A spatial variance is determined between the representative compositions. When the spatial variance falls within a predetermined spatial tolerance, the same unique identifier is assigned to the reservoired hydrocarbons. In this way, independent hydrocarbon seeps can be identified as originating from the same or different reservoir.

While preferred embodiments of the present invention have been described, it should be understood that various changes, adaptations and modifications can be made therein within the scope of the invention(s) as claimed below.

## Claims

1. A method for determining a presence of a reservoired hydrocarbons having a hydrocarbon seep, the method comprising the steps of:
a) locating a hydrocarbon seep (S1, S2, S3, S4, S5) at a seabed (28) location where hydrocarbon is actively flowing out of the seabed (28);
**characterised in that** the method further comprises:
b) determining at least two temporally spaced molecular compositions of the hydrocarbon seep (S1, S2, S3, S4, S5);
c) determining a temporal variance between the at least two temporally spaced molecular compositions;
d) when the temporal variance falls within a predetermined temporal tolerance, classifying the hydrocarbon seep (S1, S2, S3, S4, S5) as being indicative of the presence of a reservoired hydrocarbons; and
e) assigning a unique identifier to the reservoired hydrocarbons.

2. The method of claim 1, wherein step b) further comprises determining at least two temporally spaced isotopic compositions of the hydrocarbon seep (S1, S2, S3, S4, S5).

3. The method of claim 2, wherein the at least two temporally spaced isotopic compositions are selected from the group consisting of isotopologues of methane, ethane, propane, butane, carbon dioxide, hydrogen sulphide, and combinations thereof.

4. The method of claim 1, wherein the at least two temporally spaced molecular compositions comprise components selected from the group consisting of methane, ethane, propane, butane, carbon dioxide, hydrogen sulphide, and combinations thereof.

5. The method of claim 1, wherein step b) is determined in-situ, remotely or a combination thereof.

6. The method of claim 1, wherein a plurality of hydrocarbon seeps (S1, S2, S3, S4, S5) are detected in step a) and wherein at least two temporally spaced molecular compositions are determined in step b) for each of the plurality of hydrocarbon seeps (S1, S2, S3, S4, S5).

7. The method of claim 6, further comprising the steps of:
f) selecting a representative molecular composition for each of the plurality of hydrocarbon seeps (S1, S2, S3, S4, S5) classified as being indicative of the presence of reservoired hydrocarbons;
g) determining a spatial variance between the representative molecular compositions; and
h) when the spatial variance falls within a predetermined spatial tolerance, assigning the same unique identifier to the reservoired hydrocarbons.

8. A method for determining a unique hydrocarbon reservoir having a hydrocarbon seep, the method comprising the steps of:
a) locating a plurality of hydrocarbon seeps (S1, S2, S3, S4, S5) at a seabed (28) location where hydrocarbon is actively flowing out of the seabed (28);
**characterised in that** the method further comprises:
b) determining at least two temporally spaced molecular compositions for each of the plurality of hydrocarbon seeps (S1, S2, S3, S4, S5);
c) determining a temporal variance between the at least two temporally spaced molecular compositions for each of the plurality of hydrocarbon seeps (S1, S2, S3, S4, S5);
d) when the temporal variance for any one of the plurality of hydrocarbon seeps (S1, S2, S3, S4, S5) falls within a predetermined temporal tolerance, classifying the hydrocarbon seep (S1, S2, S3, S4, S5) as being indicative of a presence of reservoired hydrocarbons;
e) selecting a representative molecular composition for each of the plurality of hydrocarbon seeps (S1, S2, S3, S4, S5) classified as being indicative of the presence of reservoired hydrocarbons;
f) determining a spatial variance between the representative molecular compositions;
g) for the hydrocarbon seeps (S1, S2, S3, S4, S5) having the spatial variance falling within a predetermined spatial tolerance, assigning a unique identifier to the reservoired hydrocarbons; and
h) for the one or more hydrocarbon seeps (S1, S2, S3, S4, S5) wherein the spatial variance falls outside the predetermined spatial tolerance, assigning one or more additional unique identifiers to the reservoired hydrocarbons.

9. The method of claim 8, wherein step b) further comprises determining at least temporally spaced two isotopic compositions for each of the plurality of hydrocarbon seeps (S1, S2, S3, S4, S5).

10. The method of claim 9. wherein the at least two temporally spaced isotopic compositions are selected from the group consisting of isotopologues of methane, ethane, propane, butane, carbon dioxide, hydrogen sulphide, and combinations thereof.

11. The method of claim 8, wherein step b) is determined in-situ, remotely or a combination thereof.

## Patentansprüche

1. Verfahren zum Bestimmen eines Vorhandenseins von eingelagerten Kohlenwasserstoffen, die eine Kohlenwasserstoffsickerstelle aufweisen, das Verfahren umfassend die Schritte:
a) Lokalisieren einer Kohlenwasserstoffsickerstelle (S1, S2, S3, S4, S5) an einer Position eines Meeresbodens (28), wo Kohlenwasserstoff aus dem Meeresboden (28) aktiv ausströmt;
**dadurch gekennzeichnet, dass** das Verfahren ferner umfasst:
b) Bestimmen mindestens zweier zeitlich beabstandeter Molekülzusammensetzungen der Kohlenwasserstoffsickerstelle (S1, S2, S3, S4, S5);
c) Bestimmen einer zeitlichen Varianz zwischen den mindestens zwei zeitlich beabstandeten Molekülzusammensetzungen;
d) wenn die zeitliche Varianz innerhalb einer zuvor bestimmten zeitlichen Toleranz fällt, Klassifizieren des Kohlenwasserstoffsickerstelle (S1, S2, S3, S4, S5) als hinweisend auf das Vorhandensein eines eingelagerten Kohlenwasserstoffs; und
e) Zuweisen eines eindeutigen Bezeichners zu den eingelagerten Kohlenwasserstoffen.

2. Verfahren nach Anspruch 1, wobei Schritt b) ferner das Bestimmen mindestens zweier zeitlich beabstandeter Isotopenzusammensetzungen der Kohlenwasserstoffsickerstelle (S1, S2, S3, S4, S5) umfasst.

3. Verfahren nach Anspruch 2, wobei die mindestens zwei zeitlich beabstandeten Isotopenzusammensetzungen aus der Gruppe ausgewählt sind, bestehend aus Isotopologen von Methan, Ethan, Propan, Butan, Kohlendioxid, Schwefelwasserstoff und Kombinationen davon.

4. Verfahren nach Anspruch 1, wobei die mindestens zwei zeitlich beabstandeten Molekülzusammensetzungen Komponenten umfassen, die aus der Gruppe ausgewählt sind, bestehend aus Methan, Ethan, Propan, Butan, Kohlendioxid, Schwefelwasserstoff und Kombinationen davon.

5. Verfahren nach Anspruch 1, wobei Schritt b) vor Ort, aus der Ferne oder durch eine Kombination davon bestimmt wird.

6. Verfahren nach Anspruch 1, wobei in Schritt a) eine Vielzahl von Kohlenwasserstoffsickerstellen (S1, S2, S3, S4, S5) erkannt werden, und wobei in Schritt b) für jede der Vielzahl von Kohlenwasserstoffsickerstellen (S1, S2, S3, S4, S5) mindestens zwei zeitlich beabstandete Molekülzusammensetzungen bestimmt werden.

7. Verfahren nach Anspruch 6, ferner umfassend die Schritte:
f) Auswählen einer repräsentativen Molekülzusammensetzung für jede der Vielzahl von Kohlenwasserstoffsickerstellen (S1, S2, S3, S4, S5), die als hinweisend auf das Vorhandensein von eingelagerten Kohlenwasserstoffen klassifiziert sind;
g) Bestimmen einer räumlichen Varianz zwischen den repräsentativen Molekülzusammensetzungen; und
h) wenn die räumliche Varianz innerhalb einer zuvor bestimmten räumlichen Toleranz fällt, Zuweisen desselben eindeutigen Bezeichners zu den eingelagerten Kohlenwasserstoffen.

8. Verfahren zum Bestimmen einer eindeutigen Kohlenwasserstofflagerstätte, die eine Kohlenwasserstoffsickerstelle aufweist, das Verfahren umfassend die Schritte:
a) Lokalisieren einer Vielzahl von Kohlenwasserstoffsickerstellen (S1, S2, S3, S4, S5) an der Position eines Meeresbodens (28), wo Kohlenwasserstoff aus dem Meeresboden (28) aktiv ausströmt;
**dadurch gekennzeichnet, dass** das Verfahren ferner umfasst:
b) Bestimmen mindestens zweier zeitlich beabstandeter Molekülzusammensetzungen für jede der Vielzahl von Kohlenwasserstoffsickerstellen (S1, S2, S3, S4, S5);
c) Bestimmen einer zeitlichen Varianz zwischen den mindestens zwei zeitlich beabstandeten Molekülzusammensetzungen für jede der Vielzahl von Kohlenwasserstoffsickerstellen (S1, S2, S3, S4, S5);
d) wenn die zeitliche Varianz für eine beliebige der Vielzahl von Kohlenwasserstoffsickerstellen (S1, S2, S3, S4, S5) innerhalb einer zuvor bestimmten zeitlichen Toleranz fällt, Klassifizieren der Kohlenwasserstoffsickerstelle (S1, S2, S3, S4, S5) als hinweisend auf ein Vorhandensein von eingelagerten Kohlenwasserstoffen;
e) Auswählen einer repräsentativen Molekülzusammensetzung für jede der Vielzahl von Kohlenwasserstoffsickerstellen (S1, S2, S3, S4, S5), die als hinweisend auf das Vorhandensein von eingelagerten Kohlenwasserstoffen klassifiziert sind;
f) Bestimmen einer räumlichen Varianz zwischen den repräsentativen Molekülzusammensetzungen;
g) für die Kohlenwasserstoffsickerstellen (S1, S2, S3, S4, S5), die die räumliche Varianz aufweisen, die innerhalb einer zuvor bestimmten räumlichen Toleranz fällt, Zuweisen eines eindeutigen Bezeichners zu den eingelagerten Kohlenwasserstoffen; und
h) für die eine oder die mehreren Kohlenwasserstoffsickerstellen (S1, S2, S3, S4, S5), wobei die räumliche Varianz außerhalb der zuvor bestimmten räumlichen Toleranz fällt, Zuweisen eines oder mehrerer zusätzlicher eindeutiger Bezeichner zu den eingelagerten Kohlenwasserstoffen.

9. Verfahren nach Anspruch 8, wobei Schritt b) ferner das Bestimmen mindestens zweier zeitlich beabstandeter Isotopenzusammensetzungen für jede der Vielzahl von Kohlenwasserstoffsickerstellen (S1, S2, S3, S4, S5) umfasst.

10. Verfahren nach Anspruch 9, wobei die mindestens zwei zeitlich beabstandeten Isotopenzusammensetzungen aus der Gruppe ausgewählt sind, bestehend aus Isotopologen von Methan, Ethan, Propan, Butan, Kohlendioxid, Schwefelwasserstoff und Kombinationen davon.

11. Verfahren nach Anspruch 8, wobei Schritt b) vor Ort, aus der Ferne oder durch eine Kombination davon bestimmt wird.

## Revendications

1. Procédé de détermination de la présence d'hydrocarbures de gisement présentant un suintement d'hydrocarbures, le procédé comprenant les étapes suivantes :
a) la localisation d'un suintement
d'hydrocarbures (S1, S2, S3, S4, S5) au niveau d'un emplacement du fond marin (28) où des hydrocarbures s'écoulent activement hors du fond marin (28) ; **caractérisé en ce que** le procédé consiste en outre à :
b) la détermination d'au moins deux compositions moléculaires espacées dans le temps du suintement d'hydrocarbures (S1, S2, S3, S4, S5) ;
c) la détermination d'une variance temporelle entre les au moins deux compositions moléculaires espacées dans le temps ;
d) lorsque la variance temporelle s'inscrit dans une tolérance temporelle prédéterminée, le classement du suintement d'hydrocarbures (S1, S2, S3, S4, S5) comme indiquant la présence d'hydrocarbures de gisement ; et
e) l'attribution d'un identifiant unique aux hydrocarbures de gisement.

2. Procédé selon la revendication 1, dans lequel l'étape b) comprend en outre la détermination d'au moins deux compositions isotopiques espacées dans le temps du suintement d'hydrocarbures (S1, S2, S3, S4, S5) .

3. Procédé selon la revendication 2, dans lequel les au moins deux compositions isotopiques espacées dans le temps sont choisies dans le groupe constitué par des isotopologues du méthane, de l'éthane, du propane, du butane, du dioxyde de carbone, du sulfure d'hydrogène, et des combinaisons de ceux-ci.

4. Procédé selon la revendication 1, dans lequel les au moins deux compositions moléculaires espacées dans le temps comprennent des composants choisis dans le groupe constitué par le méthane, l'éthane, le propane, le butane, le dioxyde de carbone, le sulfure d'hydrogène et des combinaisons de ceux-ci.

5. Procédé selon la revendication 1, dans lequel l'étape b) est déterminée in situ, à distance ou une combinaison de ceux-ci.

6. Procédé selon la revendication 1, dans lequel une pluralité de suintements d'hydrocarbures (S1, S2, S3, S4, S5) sont détectés à l'étape a) et dans lequel au moins deux compositions moléculaires espacées dans le temps sont déterminées à l'étape b) pour chaque suintement de la pluralité de suintements d'hydrocarbures (S1, S2, S3, S4, S5).

7. Procédé selon la revendication 6, comprenant en outre les étapes consistant à :
f) sélectionner une composition moléculaire représentative pour chaque suintement de la pluralité de suintements d'hydrocarbures (S1, S2, S3, S4, S5) classés comme indiquant la présence d'hydrocarbures de gisement ;
g) déterminer une variance spatiale entre les compositions moléculaires représentatives ; et
h) lorsque la variance spatiale s'inscrit dans une tolérance spatiale prédéterminée, attribuer le même identifiant unique aux hydrocarbures de gisement.

8. Procédé de détermination d'un gisement unique d'hydrocarbures présentant un suintement d'hydrocarbures, le procédé comprenant les étapes suivantes :
a) la localisation d'une pluralité de suintements d'hydrocarbures (S1, S2, S3, S4, S5) au niveau d'un emplacement du fond marin (28) où des hydrocarbures s'écoulent activement hors du fond marin (28) ;
**caractérisé en ce que** le procédé consiste en outre à :
b) la détermination d'au moins deux compositions moléculaires espacées dans le temps pour chaque suintement de la pluralité de suintements d'hydrocarbures (S1, S2, S3, S4, S5) ;
c) la détermination d'une variance temporelle entre les au moins deux compositions moléculaires espacées dans le temps pour chaque suintement de la pluralité de suintements d'hydrocarbures (S1, S2, S3, S4, S5) ;
d) lorsque la variance temporelle pour un quelconque suintement de la pluralité de suintements d'hydrocarbures (S1, S2, S3, S4, S5) s'inscrit dans une tolérance temporelle prédéterminée, le classement du suintement d'hydrocarbures (S1, S2, S3, S4, S5) comme indiquant la présence d'hydrocarbures de gisement ;
e) la sélection d'une composition moléculaire représentative pour chaque suintement de la pluralité de suintements d'hydrocarbures (S1, S2, S3, S4, S5) classés comme indiquant la présence d'hydrocarbures de gisement ;
f) la détermination d'une variance spatiale entre les compositions moléculaires représentatives ;
g) pour les suintements d'hydrocarbures (S1, S2, S3, S4, S5) dont la variance spatiale s'inscrit dans une tolérance spatiale prédéterminée, l'attribution d'un identifiant unique aux hydrocarbures de gisement ; et
h) pour le ou les suintements d'hydrocarbures (S1, S2, S3, S4, S5) dont la variance spatiale est hors de la tolérance spatiale prédéterminée, l'attribution d'un ou plusieurs identifiants uniques supplémentaires aux hydrocarbures de gisement.

9. Procédé selon la revendication 8, dans lequel l'étape b) comprend en outre la détermination d'au moins deux compositions isotopiques espacées dans le temps pour chaque suintement de la pluralité de suintements d'hydrocarbures (S1, S2, S3, S4, S5).

10. Procédé selon la revendication 9, dans lequel les au moins deux compositions isotopiques espacées dans le temps sont choisies dans le groupe constitué par des isotopologues du méthane, de l'éthane, du propane, du butane, du dioxyde de carbone, du sulfure d'hydrogène, et des combinaisons de ceux-ci.

11. Procédé selon la revendication 8, dans lequel l'étape b) est déterminée in situ, à distance ou une combinaison de ceux-ci.
